# EUROPEAN PATENT APPLICATION

(11) **EP 4 268 828 A1**
(43) Date of publication of application: **01.11.2023**
(21) Application number: 21915185.9
(22) Date of filing: 23.12.2021
(51) Int. Cl.: A61K 31/7016, A61K 8/44, A61K 8/60, A61K 8/73, A61K 31/702, A61K 47/18, A61Q 11/00

(54) **COMPOSITION FOR ORAL CAVITY**

(30) Priority: 28.12.2020 JP 2020218922
(71) Applicant: Sunstar Suisse SA, 1163 Etoy (CH)
(72) Inventor: AKASE Takanori, Takatsuki-shi, Osaka 569-1195 (JP); SUZUKI Takuma, Takatsuki-shi, Osaka 569-1195 (JP)
(74) Representative: Murgitroyd & Company
(86) International application number: PCT/JP2021/047772
(87) International publication number: WO 2022/145325

(57) **Abstract**

This composition for the oral cavity contains an amino acid and a specific sugar. The specific sugar is at least one selected from lactose, 2'-fucosyllactose, galactose, raffinose, and a galactooligosaccharide.

## Description

### TECHNICAL FIELD

The present invention relates to a composition for the oral cavity.

### BACKGROUND ART

Bacteria in the oral cavity are mostly free-floating or present in a bacterial aggregate called biofilm. Since the biofilm is resistant to physical and chemical actions, removal of the biofilm is more difficult than removal of the floating bacteria.

The mechanism of how bacteria floating in the oral cavity adhere to the surfaces of teeth and the oral mucous membrane has long been studied and various findings have been obtained. To inhibit formation of a biofilm effectively, it is considered important to prevent adherence of early colonizers to intraoral tissue. Early colonizers are bacteria that quickly adhere to a clean tooth surface to form initial plaque. It is known that the early colonizers have receptors that are bound to salivary proteins, and that bacterial adherence is promoted by binding of the receptors to specific salivary proteins. The salivary proteins are roughly classified into a proline-rich protein group and a proline-rich glycoprotein group. As typical bacteria having a receptor for the former group of salivary proteins, for example, *Streptococcus mutans, Streptococcus gordonii,* and *Porphyromonas gingivalis* are known. As typical bacteria having a receptor for the latter group of salivary proteins, for example, *Staphylococcus aureus, Fusobacterium nucleatum,* and *Streptococcus oralis* are known. A technique has been needed for preventing adherence of bacteria belonging to these two groups effectively. For example, Patent Literature 1 discloses a technique for preventing bacteria such as *Streptococcus mutans* and *Staphylococcus aureus* from adhering to intraoral tissue and proliferating, by the use of a composition containing a liposome having a hydrogenated phospholipid as an essential component.

### CITATION LIST

### Patent Literature

Patent Literature 1: Japanese Patent Laid-Open No. 2018-115179

### SUMMARY OF INVENTION

### Technical Problem

As a result of inventive studies by the present inventors, it was found that the concentration of *Streptococcus oralis* (hereinafter referred to as *S*. *oralis*) in persons having no history of dental caries is higher than that in persons having a history of dental caries. This finding suggests that, in order to keep a healthy oral environment, it is important to increase the concentration of *S*. *oralis* in the oral cavity.

The present invention has been attained based on the above finding. An object of the invention is to increase the concentration of *S*. *oralis* in the oral cavity.

### Solution to Problem

The composition for the oral cavity for attaining the aforementioned object is a composition for the oral cavity containing an amino acid and a sugar, in which the sugar is at least one selected from lactose, 2'-fucosyllactose, galactose, raffinose, and galacto-oligosaccharide.

In the composition for the oral cavity, the amino acid can be at least one selected from an acidic amino acid and an acidic amino acid salt.

### DESCRIPTION OF EMBODIMENTS

Now, an embodiment of the present invention will be described.

A composition for the oral cavity according to the embodiment contains a specific sugar and an amino acid.

### Specific Sugar

The specific sugar contained in the composition for the oral cavity is at least one selected from lactose, 2'-fucosyllactose, galactose, raffinose, galacto-oligosaccharide, and hydrates of these. The composition for the oral cavity may contain only one or a combination of two types or more of the specific sugars mentioned above.

The content of the specific sugar in the composition for the oral cavity is, for example, 0.5 to 4 % by mass, preferably 1 to 3 % by mass, and more preferably 1 to 2 % by mass.

### Amino Acid

The amino acid contained in the composition for the oral cavity is not particularly limited. Examples of the amino acid include neutral amino acids such as alanine, proline, threonine, serine, valine, glycine, and citrulline; acidic amino acids such as aspartic acid and glutamic acid, and acidic amino acid salts; and basic amino acids such as lysine, arginine, and histidine, and basic amino acid salts. Examples of the acidic amino acid salts include alkali metal salts such as a sodium salt and a potassium salt; alkaline earth metal salts such as a magnesium salt and a calcium salt. Examples of the basic amino acid salts include a hydrochloride and a sulfate.

The composition for the oral cavity may contain only one or a combination of two types or more of the amino acids mentioned above. The composition for the oral cavity particularly preferably contains at least one selected from acidic amino acids and acidic amino acid salts, as the amino acid.

The content of the amino acid in the composition for the oral cavity is, for example, 0.5 to 4 % by mass, preferably 1 to 3 % by mass, and more preferably 1.5 to 3 % by mass. The mass ratio of the amino acid to the specific sugar in the composition for the oral cavity (amino acid/specific sugar) is, for example, 0.25 to 4, preferably 0.5 to 3, and more preferably 1 to 2.

### Combination of Specific Sugar and Specific Amino Acid

The composition for the oral cavity preferably contains at least one selected from lactose, galactose, raffinose, and galacto-oligosaccharide, as the specific sugar, and at least one selected from aspartic acid and aspartate, as the specific amino acid. The composition for the oral cavity preferably contains at least one selected from raffinose and galacto-oligosaccharide, as the specific sugar, and at least one selected from glutamic acid and glutamate, as the specific amino acid. The composition for the oral cavity preferably contains galactose, as the specific sugar, and citrulline, as the specific amino acid. The composition for the oral cavity preferably contains 2'-fucosyllactose, as the specific sugar, and arginine, as the specific amino acid.

### Application Form, Use, and Dosage Form

The application form of the composition for the oral cavity is not particularly limited, and the composition can be used, for example, as pharmaceuticals, quasi-drugs, and cosmetics. The composition for the oral cavity can be appropriately used in common products, such as a toothpaste, a mouthwash, a gargle, a liquid toothpaste, a biofilm dispersant, a bad breath preventive agent, a gum massage agent, a moisturizing agent for the oral cavity, a tongue coating remover, an intraoral liniment, an oral disinfectant, a throat disinfectant, an agent for the oral cavity/throat, a periodontal disease therapeutic agent, a denture liner, a denture coating agent, a denture adhesive, a denture keeper, a denture cleanser, and an implant care agent.

The dosage form of the composition for the oral cavity is not particularly limited, and the composition containing a solvent such as water and an alcohol can be employed as, for example, an ointment, a paste, a dermatological paste, a spray agent, a gel agent, a liquid, a suspension/emulsification agent, and a gum.

The type of water to be used as a solvent is not particularly limited, and, for example, distilled water, pure water, ultrapure water, purified water, and tap water can be used. The type of alcohol to be used as a solvent is not particularly limited, and, for example, ethanol can be used. Water and an alcohol can be used as a mixture.

In the case in which a composition for the oral cavity is prepared in the form of a liquid, the content of a solvent such as water is preferably 60 to 99.8 % by mass, and more preferably 70 to 90 % by mass.

The composition for the oral cavity can be used as a bacterial-proliferation promoting composition for stimulating proliferation of *S*. *oralis* in the oral cavity. Examples of the bacterial-proliferation promoting composition include foods and drinks, as well as pharmaceuticals, quasi-drugs, and cosmetics as mentioned above.

The composition for the oral cavity to be used as foods and drinks can be used as a material for various foods or can be used by being added to beverage materials. The form of the foods and drinks is not particularly limited, and the form may be any one of a liquid, a powder, a gel, and a solid form. Examples of the foods and drinks include a chewable tablet, a troche, a tablet, a granule, a powder, a powder for drink, a beverage, an orally disintegrating film, an oral spray, and confectioneries such as a chewing gum, a candy, a gummy candy, and a jelly. The use of the foods and drinks is not particularly limited, and the foods and drinks can be used as so-called general foods, healthy foods, functional foods, dietary supplements, supplements, foods for specified health use, foods with functional claims, and patient foods.

### Other Components

The composition for the oral cavity may contain components other than the components mentioned above, depending on the application purpose, form, and usage. Examples of the other components include an antibacterial agent, an anti-inflammatory agent, a flavoring, a moistening agent, a surfactant, an abrasive, an alcohol, a thickener, a sweetener, a medicinal component, a coloring agent, a stabilizer, and a pH adjuster. As the other components, those commonly known and blended in compositions for the oral cavity can be used. With respect to each of the other components to be contained in the composition for the oral cavity, a single type may be used alone or two or more types may be used in combination.

Examples of the antibacterial agent include cetylpyridinium chloride, paraben, sodium benzoate, triclosan, chlorhexidine hydrochloride, isopropyl methylphenol, benzalkonium chloride, benzethonium chloride, and hinokitiol.

Examples of the anti-inflammatory agent include glycyrrhizinate, tranexamic acid, ε-aminocaproic acid, and a cork tree bark extract.

Examples of the flavoring include anethole, eugenol, carvone, wintergreen, methyl salicylate, thymol, clove oil, sage oil, ocimene oil, and citronellol.

Examples of the surfactant include a nonionic surfactant, an anionic surfactant, and an amphoteric surfactant.

Examples of the nonionic surfactant include sugar fatty acid esters such as sucrose fatty acid ester and maltose fatty acid ester; sugar alcohol fatty acid esters such as maltitol fatty acid ester; sorbitan fatty acid esters such as sorbitan monolaurate; polyoxyethylene sorbitan fatty acid esters such as polyoxyethylene sorbitan monolaurate and polyoxyethylene sorbitan monostearate; fatty acid alkanolamides such as lauric acid diethanolamide; polyoxyethylene alkyl ethers such as polyoxyethylene stearyl ether and polyoxyethylene oleyl ether; polyethylene glycol fatty acid esters such as polyethylene glycol monooleate, polyethylene glycol monolaurate; alkyl glucosides such as lauryl glucoside and decyl glucoside; polyglycerin fatty acid ester; polyoxyethylene glycerin fatty acid ester; polyoxyethylene fatty acid ester; alkyl glucosides; polyoxyethylene hydrogenated castor oil; glycerin fatty acid ester; and polyoxyethylene propylene block copolymer.

Examples of the anionic surfactant include sulfates such as sodium lauryl sulfate and sodium polyoxyethylene lauryl ether sulfate; sulfosuccinates such as sodium lauryl sulfosuccinate and sodium polyoxyethylene lauryl ether sulfosuccinate; acyl amino acid salts such as sodium cocoyl sarcosinate and sodium lauroyl methylaminopropionate; and sodium methyl cocoyl taurate.

Examples of the amphoteric surfactant include amino acid-derived amphoteric surfactants such as N-lauryldiaminoethylglycine and N-myristyldiethylglycine; and betaine amphoteric surfactants such as an alkyldimethylamino acetic acid betaine, an N-alkyl-N'-carboxymethyl-N'-hydroxyethylethylenediamine salt, and a 2-alkyl-N-carboxymethyl-N-hydroxyethylimidazolinium betaine.

Examples of the abrasive include calcium carbonate, magnesium carbonate, dibasic calcium phosphate, tribasic calcium phosphate, magnesium phosphate, silica, zeolite, sodium metaphosphate, aluminum hydroxide, magnesium hydroxide, calcium pyrophosphate, red iron oxide, calcium sulfate, and silicic anhydride.

Examples of the alcohol include ethyl alcohol, lauryl alcohol, and myristyl alcohol.

Examples of the thickener include sodium polyacrylate, carrageenan, carboxymethylcellulose sodium, sodium alginate, xanthan gum, hydroxyethyl cellulose, hydroxypropyl methylcellulose, methyl cellulose, and alginate propylene glycol ester.

Examples of the medicinal component include fluorides such as sodium monofluorophosphate, sodium fluoride, stannous fluoride, and strontium fluoride; condensed phosphates such as sodium pyrophosphate and sodium polyphosphate; phosphates such as sodium monohydrogen phosphate and trisodium phosphate; vitamin preparations such as ascorbic acid, sodium ascorbate, pyridoxine hydrochloride, and tocopherol acetate; glucanase enzymes such as dextranase and mutanase; degrading enzymes such as protease and lysozyme; inorganic salts such as zinc chloride, zinc citrate, strontium chloride, and potassium nitrate; chelation compounds such as chlorophyll and glycerophosphate; polyethylene glycols and the like for dissolving fats; sodium chloride; aluminum lactate; and strontium chloride.

Examples of the coloring agent include certified colors such as green No. 1, blue No. 1, and yellow No. 4, and titanium oxide.

Examples of the stabilizer include sodium edetate, sodium thiosulfate, sodium sulfite, calcium lactate, lanolin, triacetin, castor oil, and magnesium sulfate.

Examples of the pH adjuster include citric acid, malic acid, lactic acid, tartaric acid, acetic acid, phosphoric acid, pyrophosphoric acid, glycerophosphoric acid, and salts of these such as potassium salts, sodium salts and ammonium salts, and sodium hydroxide. The composition for the oral cavity preferably contains a pH adjuster to adjust pH to fall in the range of 4 to 9, and particularly 5 to 7.

Now, the action according to the embodiment will be described.

Although the details are omitted, when the concentration of *S*. *oralis* in the saliva was compared between a group of subjects having a history of dental caries and a group of subjects not having a history of dental caries, the concentration *of S. oralis* in the group of subjects not having a history of dental caries was significantly higher. From the result, it is considered important to increase the concentration *of S. oralis* in the oral cavity in order to keep a healthy oral environment. The mechanism is estimated as follows:

The early colonizers, which are bacteria adhering to a tooth surface to form initial plaque, mediate adherence of pathogenic bacteria forming a biofilm to the tooth surface. Of the early colonizers, *S*. *oralis* has a poor ability to adhere to biofilm-forming pathogenic bacteria. The pathogenic bacteria rarely adhere to the tooth-surface region having *S*. *oralis* adhered, compared to regions having other early colonizers adhered.

Because of this, an increase in concentration of *S*. *oralis* in the oral cavity increases the proportion of the tooth-surface region having *S*. *oralis* adhered in the tooth-surface region having early colonizers adhered. In other words, an increase in concentration of *S*. *oralis* in the oral cavity competitively inhibits adhesion to a tooth surface of other early colonizers to which pathogenic bacteria is easily adhere. As a result of suppressing adhesion to a tooth surface of other early colonizers to which pathogenic bacteria is easily adhere, formation of a biofilm is suppressed, with the result that a healthy oral environment can be easily obtained.

The composition for the oral cavity of the embodiment can proliferate *S*. *oralis* in the oral cavity. It is expected that the composition for the oral cavity of the embodiment may produce an effect of suppressing biofilm formation based on the effect of proliferating *S*. *oralis* in the oral cavity.

Now, the advantages of the embodiment will be described.
(1) The composition for the oral cavity contains an amino acid and a specific sugar. The specific sugar is at least one selected from lactose, 2'-fucosyllactose, galactose, raffinose and galacto-oligosaccharide. According to the configuration, it is possible to proliferate *S*. *oralis* in the oral cavity.
(2) The amino acid is at least one selected from an acidic amino acid and an acidic amino acid salt. In this case, the effect of proliferating *S*. *oralis* in the oral cavity is promoted.
(3) The specific sugar is at least one selected from lactose, galactose, raffinose and galacto-oligosaccharide. The amino acid is at least one selected from aspartic acid and aspartate. In this case, the effect of proliferating *S*. *oralis* in the oral cavity is further promoted.
(4) The specific sugar is at least one selected from raffinose and galacto-oligosaccharide. The amino acid is at least one selected from glutamic acid and glutamate. In this case, the effect of proliferating *S*. *oralis* in the oral cavity is further promoted.

### Examples

The composition for the oral cavity of the present invention will be more specifically described by way of the following Examples and Comparative Examples. The present invention is not limited to the configurations of the following Examples.

### <Test 1>

### S. oralis culture test

*S*. *oralis* was cultured in BHI medium (Brain Heart Infusion broth (manufactured by BD)). After the turbidity of the medium at a wavelength of 660 nm was measured, the culture solution was diluted with BHI medium until the turbidity was 0.1. The diluted medium was used as a bacterial solution. As *S. oralis, Streptococcus oralis spp. tigurinus* was used.

Sample solutions of Examples and Comparative Examples were prepared by dissolving an amino acid and a sugar in the BHI medium. The types of amino acids and sugars used in the sample solutions are as shown in Table 1. The concentrations of the amino acid in the sample solutions were set at 0 mg/ml or 15 mg/ml and the concentrations of the sugar were set at 20 mg/ml.

To the wells of a 96-well plate, the bacterial solution (100 µl) and a sample solution (100 µl) were added. The 96-well plate was subjected to culturing performed at 37°C in anaerobic conditions for 24 hours. After completion of the culture, the supernatant was removed and 110 µl of an Alamar Blue solution (manufactured by Invitrogen) diluted 11-fold with phosphate buffer saline (PBS), was added. Fluorescence intensity was measured at an excitation wavelength of 545 nm and a fluorescence wavelength of 590 nm. A relative value thereof to the fluorescence intensity of a control containing no sample solution was calculated as the proliferation rate of *S. oralis.* The proliferation rate calculated was evaluated based on the following evaluation criteria. The results are shown in Table 1.

A: 10 or more
B: 5 or more and less than 10
C: less than 5

**[Table 1]**

| | Sugar | Amino acid | Evaluation of proliferation rate |
|---|---|---|---|
| Example 1 | Lactose monohydrate | Arginine | A |
| Example 2 | Galactose | | A |
| Example 3 | Raffinose | | A |
| Example 4 | Galacto-oligosaccharide | | A |
| Comparative Example 1 | - | | C |
| Comparative Example 2 | Isomalto-oligosaccharide | | C |
| Comparative Example 3 | Fructo-oligosaccharide | | C |
| Comparative Example 4 | Inulin | | C |
| Comparative Example 5 | Xylitol | | C |
| Comparative Example 6 | Erythritol | | C |
| Comparative Example 7 | Glycerin | | C |

As shown in Table 1, when the sample solutions of Examples 1 to 4, containing lactose monohydrate, galactose, raffinose, and galacto-oligosaccharide, respectively, as the sugar, and arginine as the amino acid, were added, the proliferation of *S*. *oralis* was promoted. In contrast, when the sample solution of Comparative Example 1, containing no sugar, and the sample solutions of Comparative Examples 2 to 7, containing isomalto-oligosaccharide, fructo-oligosaccharide, inulin, xylitol, erythritol and glycerin, respectively, as the sugar, were added, an effect of promoting proliferation of *S. oralis* was not obtained.

### <Test 2>

The proliferation rates of *S. oralis* were measured by using sample solutions containing lactose monohydrate, galactose, raffinose, and galacto-oligosaccharide exhibiting a proliferation promotion effect in Test 1 in combination with a different type of amino acid. The test method was the same as in Test 1. The types of amino acids and sugars used in the sample solutions and the measurement results of proliferation rates are shown in Table 2. In Test 2, the amino acid concentration was set at 15 mg/ml only in the sample solution of Example 5 where arginine was used, and set at 20 mg/ml in other Examples.

**[Table 2]**

| | Sugar | Amino acid | Proliferation rate |
|---|---|---|---|
| Example 5 | Lactose monohydrate | Arginine | 48 |
| Example 6 | | Sodium aspartate | 29.6 |
| Example 7 | | Sodium glutamate | 42.8 |
| Example 8 | | Lysine hydrochloride | 11.9 |
| Example 9 | | Alanine | 12.2 |
| Example 10 | | Proline | 9.1 |
| Example 11 | | Threonine | 9.3 |
| Example 12 | | Serine | 7.6 |
| Example 13 | | Valine | 9.8 |
| Example 14 | | Histidine | 2.1 |
| Example 15 | Galactose | Arginine | 13 |
| Example 16 | | Sodium aspartate | 382 |
| Example 17 | | Sodium glutamate | 32 |
| Example 18 | Raffinose | Arginine | 162 |
| Example 19 | | Sodium aspartate | 509 |
| Example 20 | | Sodium glutamate | 291 |
| Example 21 | Galacto-oligosaccharide | Arginine | 61 |
| Example 22 | | Sodium aspartate | 226 |
| Example 23 | | Sodium glutamate | 295 |

As shown in Table 2, in all cases in which sample solutions of Example 5 to Example 23 containing different types of amino acids were added, proliferation of *S. oralis* was promoted. An effect of promoting proliferation of *S. oralis* was particularly significant in the case in which an acidic amino acid salt, i.e., sodium aspartate or sodium glutamate was used as the amino acid. Particularly, in the case in which galactose and sodium aspartate were used in combination, the case in which raffinose and sodium aspartate or sodium glutamate were used in combination, and the case in which a galacto-oligosaccharide and sodium aspartate or sodium glutamate were used in combination, proliferation rates of 200 or more were obtained. Therefore, the proliferation of *S. oralis* was promoted even more significantly in those cases. From the results of Test 1 and Test 2, it was found that the effect of promoting proliferation of *S. oralis* is limitedly obtained in the case in which a specific sugar was used in combination with an amino acid arbitrarily selected.

### <Test 3>

The proliferation rate of *S. oralis* in the case in which galactose was used as the sugar and citrulline was used as the amino acid, was measured. The test method was the same as in Test 1. The concentrations of galactose and citrulline in sample solutions and the measurement results of proliferation rates are shown in Table 3. In Test 3, the concentrations of galactose in sample solutions were set at 0 mg/ml or 2 mg/ml and the concentrations of citrulline were set at 0.5 to 3.0 mg/ml.

**[Table 3]**

| | Galactose (mg/ml) | Citrulline (mg/ml) | Proliferation rate |
|---|---|---|---|
| Comparative Example 8 | 0 | 0.5 | 3.8 |
| | 0 | 1.0 | 2.7 |
| | 0 | 2.0 | 1.8 |
| Example 24 | 2 | 0.5 | 4.4 |
| | 2 | 1.0 | 4.7 |
| | 2 | 2.0 | 4.7 |
| | 2 | 3.0 | 6.0 |

As shown in Table 3, the sample solutions of Comparative Example 8 contained citrulline in different concentrations but not galactose. When the sample solutions of Comparative Example 8 were added, the effect of proliferating *S. oralis* was promoted but the level of the proliferation promotion effect decreased as the concentration of citrulline increased.

The sample solutions of Example 24 contained both galactose and citrulline, and more specifically, contained galactose in the same concentration and citrulline in different concentrations. When the sample solutions of Example 24 were added, the level of the proliferation promotion effect increased as the concentration of citrulline increased. When the cases containing citrulline in the same concentration were compared, the proliferation rate of any one of the sample solutions of Example 24 was higher than those of the sample solutions of Comparative Example 8.

From the above result, it was found that the use of galactose in combination with citrulline changes the effect of citrulline on proliferation of *S. oralis.* More specifically, when citrulline is used alone, the effect of promoting proliferation of *S. oralis* tends to decrease in a citrulline-concentration dependent manner. The downward tendency of the S. *oralis* proliferation promotion effect when citrulline is used alone changes to upward tendency when galactose is used in combination with citrulline; that is, proliferation is promoted in a citrulline-concentration dependent manner. This result shows that citrulline and galactose have an effect on the proliferation of *S. oralis* not individually but in a coordinated manner.

### <Test 4>

The proliferation rate of *S. oralis* was measured in the case in which 2'-fucosyllactose was used as the sugar and arginine was used as the amino acid. The test method was the same as in Test 1. The concentrations of 2'-fucosyllactose and arginine in sample solutions and the measurement results of proliferation rates are shown in Table 4. In Test 4, the concentrations of 2'-fucosyllactose in sample solutions were set at 1 to 4 mg/ml and the concentrations of citrulline were set at 0 mg/ml or 1.5 mg/ml.

**Table 4**

| | 2'-Fucosyllactose (mg/ml) | Arginine (mg/ml) | Proliferation rate |
|---|---|---|---|
| Comparative Example 9 | 1 | 0 | 1.0 |
| | 2 | 0 | 1.5 |
| | 3 | 0 | 0.9 |
| | 4 | 0 | 0.6 |
| Example 25 | 1 | 1.5 | 2.2 |
| | 2 | 1.5 | 5.0 |
| | 3 | 1.5 | 3.2 |
| | 4 | 1.5 | 3.9 |

As shown in Table 4, the sample solutions of Comparative Example 9 contain 2'-fucosyllactose but not arginine and arginine in different concentrations. When sample solutions of Comparative Example 9 were added, the effect of promoting proliferation of *S. oralis* was scarcely observed.

The sample solutions of Example 25 contain both 2'-fucosyllactose and arginine, and more specifically, contained arginine in the same concentration and 2'-fucosyllactose in different concentrations. When the sample solutions of Example 25 were added, the effect of promoting proliferation of *S. oralis* was observed. The effect of promoting proliferation of *S. oralis* varied depending on the 2'-fucosyllactose concentration. This result shows that 2'-fucosyllactose and arginine have an effect on the proliferation of *S. oralis* in a coordinated manner but arginine alone does not.

Technical concepts obtained from the above-described embodiment will now be described.
(a) The composition for the oral cavity containing at least one selected from galactose, raffinose, and galacto-oligosaccharide as the sugar and at least one selected from aspartic acid and aspartate as the amino acid.
(b) The composition for the oral cavity containing at least one selected from raffinose and galacto-oligosaccharide as the sugar and at least one selected from glutamic acid and glutamate as the amino acid.
(c) The composition for the oral cavity in which the mass ratio of the amino acid to the sugar (amino acid/sugar) is 0.5 to 4.
(d) A composition for promoting proliferation of a bacterium, *Streptococcus oralis,* containing an amino acid and a sugar, in which the sugar is at least one selected from lactose, galactose, raffinose and galacto-oligosaccharide.
(e) A method for promoting proliferation of a bacterium, *Streptococcus oralis* in saliva, characterized by adding the composition for promoting bacterial proliferation to the saliva.

## Claims

1. A composition for an oral cavity, comprising an amino acid and a sugar, wherein the sugar is at least one selected from lactose, 2'-fucosyllactose, galactose, raffinose and galacto-oligosaccharide.

2. The composition for the oral cavity according to claim 1, wherein the amino acid is at least one selected from an acidic amino acid and an acidic amino acid salt.
